# EUROPEAN PATENT APPLICATION

(11) **EP 2 860 255 A1**
(43) Date of publication of application: **15.04.2015**
(21) Application number: 13188413.2
(22) Date of filing: 14.10.2013
(51) Int. Cl.: C12N 15/88, A61K 9/51, A61K 48/00

(54) **Compositions comprising cationic and neutral lipids for transfecting nucleic acid molecules into eukaryotic cells**

(71) Applicant: Technische Universität Graz, 8010 Graz (AT); Österreichische Akademie der Wissenschaften, 1010 Wien (AT)
(72) Inventor: Karbiener, Michael, 8020 Graz (AT); Scheideler, Marcel, 8010 Graz (AT); Vonach, Caroline, 8010 Graz (AT); Prassl, Ruth, 8071 Kerscheck (AT)
(74) Representative: Sonn & Partner Patentanwälte

(57) **Abstract**

The present invention relates to compositions for transfecting nucleic acid molecules into eukaryotic cells, in particular into adipocytes, said compositions comprising a liposome nanoparticle comprising a cationic lipid and a neutral lipid and at least one nucleic acid molecule encapsulated in said liposome nanoparticle.

SEQUENCE LISTING

<110>  TECHNISCHE UNIVERSITÄT GRAZ
       ÖSTERREICHISCHE AKADEMIE DER WISSENSCHAFTEN

<120>  Compositions for transfecting nucleic acid molecules into
       eukaryotic cells

<130>  R 63287

<160>  11

<170>  PatentIn version 3.5

<210>  1
<211>  22
<212>  RNA
<213>  Artificial Sequence

<220>
<223>  miRNA

<400>  1
uucaaguaau ccaggauagg cu                                              22


<210>  2
<211>  21
<212>  RNA
<213>  Artificial Sequence

<220>
<223>  miRNA

<400>  2
uucaaguaau ucaggauagg u                                               21


<210>  3
<211>  17
<212>  RNA
<213>  Artificial Sequence

<220>
<223>  miRNA

<400>  3
uucaaguaau ucaggug                                                    17


<210>  4
<211>  22
<212>  RNA
<213>  Artificial Sequence

<220>
<223>  miRNA

<400>  4
cucaguagcc aguguagauc cu                                              22


<210>  5

<211>  23
<212>  RNA
<213>  Artificial Sequence

<220>
<223>  miRNA

<400>  5
ucaagagcaa uaacgaaaaa ugu                                              23


<210>  6
<211>  22
<212>  RNA
<213>  Artificial Sequence

<220>
<223>  miRNA

<400>  6
aacuguuugc agaggaaacu ga                                               22


<210>  7
<211>  22
<212>  RNA
<213>  Artificial Sequence

<220>
<223>  miRNA

<400>  7
uguuugcaga ggaaacugag ac                                               22


<210>  8
<211>  23
<212>  RNA
<213>  Artificial Sequence

<220>
<223>  miRNA

<400>  8
aaaagugcuu acagugcagg uag                                              23


<210>  9
<211>  23
<212>  RNA
<213>  Artificial Sequence

<220>
<223>  miRNA

<400>  9
caaagugcuu acagugcagg uag                                              23


<210>  10
<211>  23
<212>  RNA
<213>  Artificial Sequence

<220>
<223>  miRNA

<400>  10
uaaagugcuu auagugcagg uag                                               23


<210>  11
<211>  19
<212>  RNA
<213>  Artificial Sequence

<220>
<223>  miRNA

<400>  11
uguuugcaga ggaaacuga                                                    19

## Description

The present invention relates to a composition for transfecting nucleic acid molecules into eukaryotic cells, in particular adipocytes.

Nucleic acid molecules like DNA and RNA can be introduced into eukaryotic cells using various chemical, physical, lipid or biological methods.

Physical methods may include direct microinjection, electroporation and biolistic particle delivery ("gene gun"). The application of such methods is often restricted to special cell lines or tissues.

Transfection carried out with chemical methods may involve the use of calcium phosphate and DEAE (diethylethanolamine). These procedures involve co-precipitation of nucleic acid molecules with calcium phosphate crystals that are taken up by the host cells. This may result in many cases in noxious effects to most of the cells. Therefore, the transfection efficiency of such methods is rather low. DEAE-Dextran is another commonly used chemical agent for transfection.

Biological systems for nucleic acid delivery may employ, e.g., viruses. Viral transfection methods employ genetically modified viruses that are no longer pathogenic. Application of virus-mediated transfection is significantly limited by viral-related immunogenicity and the size limitation of the transgene. Such systems are highly complicated, sometimes instable and are usually not easy to use for general applications.

The most widely applied transfection methods involve liposome and in particular cationic lipids. These cationic lipids interact with the nucleic acid molecules to be transfected and promote binding of the liposome to the cells so that the nucleic acid molecules will be taken up by the cells via endocytosis. These methods are easy to use and do not require any additional lab instruments since the liposomes are simply contacted with the cells to be transformed. State of the art liposome formulations have only low cytotoxic effects and often transfection rates of more than 80 % are achieved. However, some types of eukaryotic cells, in particular adipocytes, show a relatively low transfection rate using liposome formulations known in the art.

It is an object of the present invention to provide method and means for transfecting nucleic acid molecules into eukaryotic cells, in particular adipocytes, using liposomes.

Therefore, the present invention relates to a composition for transfecting nucleic acid molecules into eukaryotic cells, in particular into adipocytes, said composition comprising a liposome nanoparticle comprising a cationic lipid (a) and a neutral lipid (b) in a molar ratio (a):(b) of 1.1:1 to 2.9:1 and at least one nucleic acid molecule encapsulated in said liposome nanoparticle. According to a preferred embodiment of the present invention the molar ratio of total lipid to the at least one nucleic acid molecule is 10:1 to 5,000:1.

It surprisingly turned out that eukaryotic cells, in particular adipocytes, can be transfected efficiently with nucleic acid molecules if said nucleic acid molecules are encapsulated into liposome nanoparticles comprising cationic and neutral lipids in a defined molar ratio. The composition according to the present invention allows efficient transfection of i.a. adipocytes in a manner that is superior to state-of-the-art methods, such as HiPerFect (e.g. disclosed in Narayanan et al. (Mol. Cancer Ther. 5 (2006), 1117-1125) aor Shah et al. (Mol. Cell. Biol. 27 (2007), 4238-4247).

The molar ratio of the cationic lipid and the neutral lipid within the liposome nanoparticle of the present invention is between 1.1:1 and 2.9:1. According to a preferred embodiment of the present invention said molar ratio can be 1.3:1 to 2.5:1, more preferably 1.4:1 to 2.4:1, even more preferably 1.5:1 to 2.3:1 or 1.6:1 to 2.2:1 or 1.7:1 to 2.1:1 or 1.8:1 to 2.1:1 or 1.9:1 to 2.1:1, in particular 2:1. The molar ratio of the aforementioned lipids can also be 1.1:1 to 2.8:1, 1.1:1 to 2.7:1, 1.1:1 to 2.6:1, 1.1:1 to 2.5:1, 1.1:1 to 2.4:1, 1.1:1 to 2.3:1, 1.1:1 to 2.2:1, 1.1:1 to 2.1:1, 1.1:1 to 2:1, 1.2:1 to 2.9:1, 1.2:1 to 2.8:1, 1.2:1 to 2.7:1, 1.2:1 to 2.6:1, 1.2:1 to 2.5:1, 1.2:1 to 2.4:1, 1.2:1 to 2.3:1, 1.2:1 to 2.2:1, 1.2:1 to 2.1:1, 1.2:1 to 2:1, 1.3:1 to 2.9:1, 1.3:1 to 2.8:1, 1.3:1 to 2.7:1, 1.3:1 to 2.6:1, 1.3:1 to 2.5:1, 1.3:1 to 2.4:1, 1.3:1 to 2.3:1, 1.3:1 to 2.2:1, 1.3:1 to 2.1:1, 1.3:1 to 2:1, 1.4:1 to 2.9:1, 1.4:1 to 2.8:1, 1.4:1 to 2.7:1, 1.4:1 to 2.6:1, 1.4:1 to 2.5:1, 1.4:1 to 2.4:1, 1.4:1 to 2.3:1, 1.4:1 to 2.2:1, 1.4:1 to 2.1:1, 1.4:1 to 2:1, 1.5:1 to 2.9:1, 1.5:1 to 2.8:1, 1.5:1 to 2.7:1, 1.5:1 1 to 2.6:1, 1.5:1 to 2.5:1, 1.5:1 to 2.4:1, 1.5:1 1 to 2.3:1, 1.5:1 to 2.2:1, 1.5:1 to 2.1:1, 1.5:1 to 2:1, 1.6:1 to 2.9:1, 1.6:1 1 to 2.8:1, 1.6:1 to 2.7:1, 1.6:1 to 2.6:1, 1.6:1 1 to 2.5:1, 1.6:1 to 2.4:1, 1.6:1 to 2.3:1, 1.6:1 to 2.2:1, 1.6:1 to 2.1:1, 1.6:1 to 2:1, 1.7:1 to 2.9:1, 1.7:1 to 2.8:1, 1.7:1 1 to 2.7:1, 1.7:1 1 to 2.6:1, 1.7:1 to 2.5:1, 1.7:1 to 2.4:1, 1.7:1 1 to 2.3:1, 1.7:1 to 2.2:1, 1.7:1 to 2.1:1, 1.7:1 to 2:1, 1.2:1 1 to 2.9:1, 1.8:1 1 to 2.8:1, 1.8:1 to 2.7:1, 1.8:1 to 2.6:1, 1.8:1 to 2.5:1, 1.8:1 to 2.4:1, 1.8:1 to 2.3:1, 1.8:1 to 2.2:1, 1.8:1 to 2.1:1, 1.8:1 to 2:1, 1.9:1 to 2.9:1, 1.9:1 to 2.8:1, 1.9:1 to 2.7:1, 1.9:1 to 2.6:1, 1.9:1 to 2.5:1, 1.9:1 to 2.4:1, 1.9:1 to 2.3:1, 1.9:1 to 2.2:1, 1.9:1 to 2.1:1 or 1.9:1 to 2:1.

Also the molar ratio of the total lipid content to the nucleic acid molecules influences the transfection rate of the nucleic acid molecules into the eukaryotic cells. At molar ratio of total lipid ((a)+(b)) to the nucleic acid molecules (c) of ((a)+(b)):(c) of 10:1 to 5,000:1 optimal transfection rates can be observed.

The molar ratio of total lipid to the at least one nucleic acid molecule is preferably 15:1 to 4,500:1, more preferably 20:1 to 4,000:1, even more preferably 50:1 to 3,000:1 or 500:1 to 2,000:1.

The liposome nanoparticles can be produced with methods known in the art, preferably using extrusion techniques or ethanol injection as described in Wagner A et al (J Drug Deliv 2011, ArtID 591325). The liposome nanoparticles of the present invention preferably have a diameter of 50 to 400 nm, more preferably of 50 to 300 nm, even more preferably of 90 to 210 nm, most preferably 100 to 200 nm.

The nucleic acid molecule of the composition of the present invention may be a DNA or RNA having a length of 10 to 500, preferably of 15 to 400, nucleotides.

The at least one nucleic acid molecule is preferably a small RNA having preferably a length of 15 to 100, preferably of 15 to 50, more preferably of 15 to 30, nucleotides.

According to a preferred embodiment of the present invention the small RNA is a small interfering RNA (siRNA) or microRNA (miRNA), preferably a miRNA. Endogenous miRNAs are generated from longer RNAs, the so-called primary miRNAs (pri-miRNAs) and precursor miRNAs (pre-miRNAs). Accordingly, also the pre-miRNAs, which are ∼80nt in size, can be efficiently transfected with the system according to the present invention. Within cells, the pre-miRNAs are recognized by certain enzymes and further processed to yield the final "mature" miRNA (∼23nt in length). It is therefore clear that the "mature" miRNA as such, but also larger RNA molecules which contain the sequence of miR-26a, b, miR-1297 etc. can efficiently be delivered with the composition according to the present invention.

According to a preferred embodiment of the present invention the cationic lipid is selected from the group consisting of 1,2-dioleoyl-3-trimethylammonium-propane (DOTAP), Dimethyldioctadecylammonium (DDAB), 1,2-di-O-octadecenyl-3-trimethylammonium-propane (DOTMA), 3ß-[N-(N',N'-dimethylaminoethane)-carbamoyl]cholesterol hydrochloride (DC-CHOL), 1,2-dioleoyl-3-dimethylammonium-propane (DODAP), cetyl trimethyl ammonium bromide (CTAB), 1,3-dioleoyloxy-2-(6-carboxy-spermyl)-propyl-amide (DOSPER) (Solodin et al. (1995) Biochem. 43:13537-13544), or 1,2-dioleoyl-sn-glycero-3-[(N-(5-amino-1-carboxy-pentyl)iminodiacetic acid)succinyl](DOGS). Preferably, the cationic lipids used in the present invention are 1,2-dioleoyl-3-trimethylammonium-propane (DOTAP), 3ß-[N-(N',N'-dimethylaminoethane)-carbamoyl]cholesterol hydrochloride DC-CHOL and 1,2-di-O-octadecenyl-3-trimethylammonium propane (DOTMA) .

The neutral lipid is preferably selected from the group consisting of dioleoylphosphatidylethanolamine (DOPE), phospholipid 1,2-diphytanoyl-sn-glycero-3-phosphoethanolamine (DPhPE), dioleoylphosphatidylcholine (DOPC), 1,2-dihexadecylphosphoethanolamine, 1,2-dilauroylphosphoethanolamine, 1,2-dilinoleoylphosphoethanolamine, 1,2-dimyristoylphosphoethanolamine, 1,2-dioleoylphosphoethanolamine, 1,2-dipalmitoylphosphoethanolamine, 1,2-distearoylphosphoethanolamine, 1-palmitoyl-2-oleoylphosphoethanolamine (POPE), 1,2-dipalmitoylphosphoethanolamine-N-[4(p-maleimidephenyl)bu tylamide], 1,2-dipalmitoylphosphoethanolamine-N-[3-(2-pyridyldithio)pro pionate], 1,2-dioleoylphosphoethanolamine-N-(succinyl) and 1,2-dipalmitoylphosphoethanolamine-N-(succinyl).
Preferably, the neutral lipid used in the present invention is DOPE.

According to a particularly preferred embodiment of the present invention the head group of the neutral lipid can be modified with maleimidomethyl-cyclohexane-carboxamide (DOPE-MCC), maleimidophenyl-butyramide (MPP-PE), N-succinyl(Succinyl PE), ethylene-glycol, N-dodecanyl, thioethanol and N-glutaryl (DOPE-GA).

According to a preferred embodiment of the present invention a sterol compound can be used. Preferably, the sterol compound used in the present invention is selected from the group consisting of cholesterol.

According to a preferred embodiment of the present invention the lipids can be coated with polyethylene glycol (PEG) more preferably N-hydroxylsuccinimide (NHS) functionalized or maleimide functionalized polyethylene glycol (Chen et al. Molecular Therapy 18 no. 9, 1650-1656 sep. 2010,).

According to a preferred embodiment of the present invention liposome formulations can be modified with protamine, hyaluronic acid, methyl-β-cyclodextrin or albumin.

According to a preferred embodiment of the present invention the composition comprises a liposome nanoparticle comprising 1,2-bis(oleoyloxy)-3-(trimethylammonium)propane (DOTAP) as a cationic lipid and dioleoylphosphatidylethanolamine (DOPE) as a neutral lipid in a molar ratio of 1.2:1 to 5:1, preferably 1.2:1 to 4:1, more preferably 1.2:1 to 3:1, even more preferably 1.5:1 to 2.5:1, in particular 2:1, and at least one nucleic acid molecule encapsulated in said liposome nanoparticle, wherein the molar ratio of total lipid to the at least one nucleic acid molecule is 10:1 to 5,000:1.

The composition of the present invention can be applied in vivo or in vitro to eukaryotic cells. If the composition is administered to an individual, intravenous, subcutaneous, intramuscular, inhalation, especially inhalation of aerosols, absorption via the mucosa of the mouth, intraperitoneal or intradermal administration is preferred.

The adipocytes of the present invention to be transfected are preferably adipocytes from white fat tissue.

The at least one nucleic acid molecule encapsulated in the liposome nanoparticle of the present invention may have various biological functions once transfected into a eukaryotic target cell. If the eukaryotic target cell is an adipocyte, in particular an adipocyte of the white adipose tissue, the at least one nucleic acid molecule may induce or upregulate the expression of UCP1 or even downregulate the transcription of said protein.

Uncoupling protein 1 (UCP1), also known as thermogenin, is an uncoupling protein found in the mitochondria of brown adipose tissue (BAT). The role of UCP1, a transmembrane protein that decreases the proton gradient generated in oxidative phosphorylation by increasing the permeability of the inner mitochondrial membrane, in obesity and other diseases associated with the metabolism is well known in the art (see e.g. Kozak LP et al, Int J Obes 32 (2008, suppl. 7):S32-S38).

According to a preferred embodiment of the present invention the at least one nucleic acid molecule of the composition of the present invention negatively interferes concomitantly and independently with at least two UCP1-suppressors and/or suppressors of the UCP1-promoting insulin signalling pathway.

The at least one nucleic acid molecule preferably hybridises to the mRNA of a UCP1-suppressor and/or a suppressor of the UCP1-promoting insulin signalling pathway, thereby inducing degradation or preventing translation of said mRNA.

Such nucleic acid molecules are known in the art (see e.g. WO 2011/138457).

According to a particularly preferred embodiment of the present invention the at least one nucleic acid molecule is selected from the group consisting of:
(i) a polynucleotide comprising the nucleotide sequence of SEQ ID No. 1;
(ii) a polynucleotide comprising the nucleotide sequence of SEQ ID No. 2;
(iii) a polynucleotide comprising the nucleotide sequence of SEQ ID No. 3;
(iv) a polynucleotide being at least 25%, 30%, 35%, 40%, 45%, 50%, 55%, 60%, 65%, 70%, 75%, 80%, 85%, 90%, 95%, 97%, 98% or 99% identical to any one of (i) to (iii); and
(v) a polynucleotide according to (iv), which comprises the nucleotide sequence of SEQ ID No. 4.

The composition of the present invention may also comprise at least one nucleic acid molecule (e.g. miRNA) selected from the group consisting of:
(i) a polynucleotide comprising the nucleotide sequence of SEQ ID NO: 9 (i.e. miR-106a);
(ii) a polynucleotide comprising the nucleotide sequence of SEQ ID NO: 10 (i.e. miR-17);
(iii) a polynucleotide comprising the nucleotide sequence of SEQ ID NO: 11 (i.e. miR-20a);
(iv) a polynucleotide which is at least 25%, 30%, 35%, 40%, 45%, 50%, 55%, 60%, 65%, 70%, 75%, 80%, 85%, 90%, 95%, 97%, 98% or 99% identical to any one of (i) to (iii); and
(v) a polynucleotide according to (iv), which comprises the nucleotide sequence of SEQ ID NO: 33 (i.e. seed sequence of SEQ ID NOs. 9 to 11),
whereby said at least one nucleotide of (iv) and/or (v) is able to induce or upregulate expression of UCP1. The above identified nucleic acid molecules are able to induce or upregulate mitochondrial function or are able to induce or upregulate energy expenditure. Summarizing, the composition of the present invention may comprise at least one nucleic acid molecules comprising or consisting of one of the nucleotide sequences selected from the group consisting of SEQ ID NO: 1 (miR-26a: UUCAAGUAAUCCAGGAUAGGCU), SEQ ID NO: 2 (miR-26b: UUCAAGUAAUUCAGGAUAGGU), SEQ ID NO: 3 (miR-1297: UUCAAGUAAUUCAGGUG), SEQ ID NO: 4 (miR- 222*: CUCAGUAGCCAGUGUAGAUCCU), SEQ ID NO: 5 (miR-335: UCAAGAGCAAUAACGAAAAAUGU), SEQ ID NO: 6 (seed sequence of SEQ ID NOs. 1 to 3: UCAAGU), SEQ ID NO: 7 (miR-452 human: A ACU GUUU GCAGAGGAAACUGA), SEQ ID NO: 8 (miR-452 murine: UGUUUGCAGAGGAAACUGAGAC), SEQ ID NO: 9 (miR- 106a: AAAAGUGCUUACAGUGCAGGUAG), SEQ ID NO: 10 (miR-17: CAAAGUGCUUACAGUGCAGGUAG), SEQ ID NO: 11 (miR-20a: UAAAGU GCUUAUAGU GCAGGUAG), SEQ ID NO: 12 (seed sequence of SEQ ID NOs. 9 to 11: AAAGUG), SEQ ID NO: 13 (consensus sequence of SEQ ID NOs. 7 and 8: UGUUUGCAGAGGAAACUGA).

Such nucleic acid molecules are described for instance in the WO 2011/138457.

According to a preferred embodiment of the present invention the composition according to present invention is used in treating or preventing diseases or disorders of the energy homeostasis or related diseases or disorders in a mammal.

The disease or disorder of the energy homeostasis is preferably selected from the group consisting of obesity, overweight, hyperglycaemia, adiposity and metabolic syndrome if the at least one nucleic acid molecule induces or upregulates the expression of UCP1.

The present invention further relates to a method of treating or preventing disorders of energy homeostasis, like obesity, overweight, adiposity, metabolic syndrome, or diseases or disorders related to energy homeostasis disorders such as diabetes (e.g., diabetes type II), hypercholesterolemia or hypertension in a subject, said method comprising administering an effective amount of a composition comprising (a) polynucleotide(s) which induce(s) or upregulate(s) expression of UCP1 such as the one disclosed herein. Such a method in accordance with the present invention may comprise the medical administration of an agent, like the herein disclosed polynucleotides, in particular a miRNA, which is able to negatively interfere concomitantly and independently with at least two UCP-1 suppressors and/or inhibitors of the UCPl-promoting insulin signalling pathway. Such molecules are provided herein and comprise, inter alia, miR/micro RNA that comprises the sequence UCAAGU (SEQ ID NO: 6), like miR-26a (SEQ ID NO: 1), miR-26b (SEQ ID NO: 2), and miR-1297 (SEQ ID NO: 3). Also other useful polynucleotides, particularly microRNAs for the medical intervention of such disorders are provided herein.

According to a further preferred embodiment of the present invention the disease or disorder of the energy homeostasis is selected from the group consisting of anorexia and cachexia if the at least one nucleic acid molecule downregulates the expression of UCP1.

Another aspect of the present invention relates to a method for transfecting eukaryotic cells, in particular adipocytes, with nucleic acid molecules comprising the step of incubating a composition according to the present invention with eukaryotic cells, in particular adipocytes.

The present invention is further illustrated by the following figures and examples without being restricted thereto.

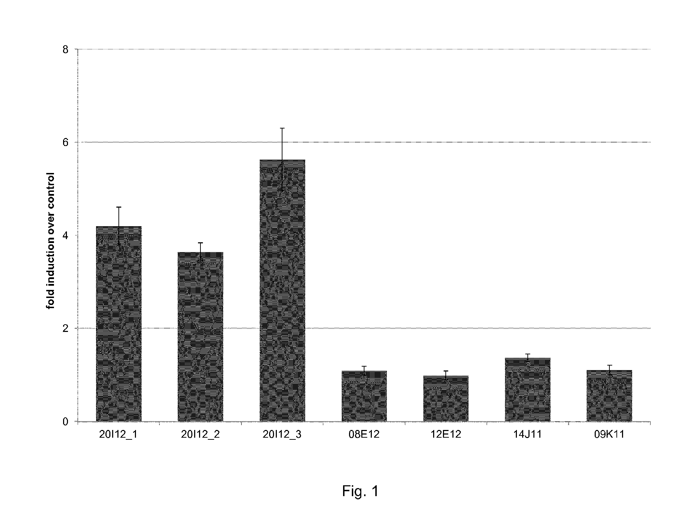

Fig. 1 shows transfection efficiency (in-fold induction over control) of transfection compositions 20I12_1, 20I12_2, 20I12_3 (according to the present invention) and 08E12, 12E12, 14J11, and 09K11 (comparative examples).

### Example 1: Preparation of Liposomes (20I12)

Liposomes composed of 1,2-dioleoyl-3-trimethylammonium-propane (chloride salt) (DOTAP) and 1,2-dioleoyl-sn-glycero-3-phosphoethanolamine (DOPE)at a 2:1 molar ratio were prepared by ethanol injection method. 3.6 mg total lipids were dissolved in chloroform to obtain a final molar ratio of DOTAP:DOPE of 2:1. The organic solvent was evaporated under a stream of nitrogen and the resulting lipid film was dried overnight in a vacuum chamber.

Thereafter the lipid film was dissolved in 100 µl ethanol and the resulting ethanol solution is injected into 900 µl deionized water.

Then the solution was sonicated in a bath-type sonicator for 2 min. Afterwards the solution was rehydrated for 1 hour at room temperature. Size measurements were performed at room temperature by dynamic light scattering (DLS) using a Zetasizer 3000HS (Malvern Instruments, Herrenburg, Germany). After size measurements the liposomes were diluted with deionized H₂O to 0.36 mg/ml lipid.

### Preparation of miRNA encapsulated cationic liposome

12 µl miR-26a (4 µM Stock) were added to 120 µl liposome solution (0.36 mg/ml) in water and mixed gently. The miRNA/liposome complexes were incubated for 15 min at room temperature and thereafter the solution was applied to the cell culture multiwell plate containing adipocytes in differentiation medium. The final miRNA concentration in a well was 22 nM.

### Example 2: Preparation of Liposomes (14J11)

Liposomes composed of 1,2-dioleoyl-3-trimethylammonium-propane (chloride salt) (DOTAP) were prepared using thin lipid film rehydration method. 2.2 mg total lipids were dissolved in chloroform. The organic solvent was evaporated under a stream of nitrogen and the resulting lipid film was dried overnight in a vacuum chamber.

Thereafter the lipid film was hydrated with 1 ml of deionized water to yield a final lipid concentration of 2.2 mg/ml.

Then the solution was sonicated in a bath-type sonicator for 2 min. Afterwards the solution was rehydrated for 1 hour at room temperature. The size of the liposomes was adjusted by extrusion through 100 nm polycarbonate membrane filter (Whatman Inc., Clifton, NJ) with a Mini-extruder (Avanti Polar Lipids, Alabaster, AL).

Size measurements were performed at room temperature by dynamic light scattering (DLS) using a Zetasizer 3000HS (Malvern Instruments, Herrenburg, Germany)

After size measurements the liposomes were diluted with deionized H₂O to 0.22 mg/ml lipid.

### Preparation of miRNA encapsulated cationic liposome

12 µl miR-26a (4 µM Stock) were added to 120 µl liposome solution (0.22 mg/ml) in water and mixed gently. The miRNA/liposome complex was incubated for 15 min at room temperature and thereafter the solution was applied to the cell culture multiwell plate containing adipocytes in differentiation medium.

### Example 3: Preparation of Liposomes (09K11)

Liposomes composed of 1,2-dioleoyl-3-trimethylammonium-propane (chloride salt) (DOTAP) were prepared using thin lipid film rehydration method. 1.1 mg total lipids were dissolved in chloroform. The organic solvent was evaporated under a stream of nitrogen and the resulting lipid film was dried overnight in a vacuum chamber.

Thereafter the lipid film was hydrated with 1 ml of deionized water to yield a final lipid concentration of 1.1 mg/ml.

Then the solution was sonicated in a bath-type sonicator for 2 min. Afterwards the solution was rehydrated for 1 hour at room temperature. The size of the liposomes was adjusted by extrusion through 100 nm polycarbonate membrane filter (Whatman Inc., Clifton, NJ) with a Mini-extruder (Avanti Polar Lipids, Alabaster, AL).

Size measurements were performed at room temperature by dynamic light scattering (DLS) using a Zetasizer 3000HS (Malvern Instruments, Herrenburg, Germany)

After size measurements the liposomes were diluted with deionized H₂O to 0.11 mg/ml lipid.

### Preparation of miRNA encapsulated cationic liposome

6 µl miR-26a (4 µM Stock) were added to 120 µl liposome solution (0.11 mg/ml) in water and mixed gently. The miRNA/liposome complex was incubated for 15 min at room temperature and thereafter the solution was applied to the cell culture multiwell plate containing adipocytes in differentiation medium.

### Example 4: Preparation of Liposomes (08E12)

Liposomes composed of 1,2-dioleoyl-3-trimethylammonium-propane (chloride salt) (DOTAP) and 1,2-dioleoyl-sn-glycero-3-phosphoethanolamine (DOPE)at a 1:1 molar ratio were prepared using thin lipid film rehydration method. 3.6 mg total lipids were dissolved in chloroform to obtain a final molar ratio of DOTAP:DOPE of 1:1. The organic solvent was evaporated under a stream of nitrogen and the resulting lipid film was dried overnight in a vacuum chamber.

Thereafter the lipid film was hydrated with 1 ml of deionized water to yield a final lipid concentration of 3.6 mg/ml.

Then the solution was sonicated in a bath-type sonicator for 2 min. Afterwards the solution was rehydrated for 1 hour at room temperature. The size of the liposomes was adjusted by extrusion through 100 nm polycarbonate membrane filter (Whatman Inc., Clifton, NJ) with a Mini-extruder (Avanti Polar Lipids, Alabaster, AL).

Size measurements were performed at room temperature by dynamic light scattering (DLS) using a Zetasizer 3000HS (Malvern Instruments, Herrenburg, Germany)

After size measurements the liposomes were diluted with deionized H₂O to 0.36 mg/ml lipid.

### Preparation of miRNA encapsulated cationic liposome

12 µl miR-26a (4 µM Stock) were added to 120 µl liposome solution (0.36 mg/ml) in water and mixed gently. The miRNA/liposome complexes were incubated for 15 min at room temperature and thereafter the solution was applied to the cell culture multiwell plate containing adipocytes in differentiation medium. The final miRNA concentration in a well was 20 nM.

### Example 5: Preparation of Liposomes (12E12)

Liposomes composed of 1,2-dioleoyl-3-trimethylammonium-propane (chloride salt) (DOTAP) and 1,2-dioleoyl-sn-glycero-3-phosphoethanolamine (DOPE)at a 3:1 molar ratio were prepared using thin lipid film rehydration method. 3.6 mg total lipids were dissolved in chloroform to obtain a final molar ratio of DOTAP:DOPE of 3:1. The organic solvent was evaporated under a stream of nitrogen and the resulting lipid film was dried overnight in a vacuum chamber.

Thereafter the lipid film was hydrated with 1 ml of deionized water to yield a final lipid concentration of 3.6 mg/ml.

Then the solution was sonicated in a bath-type sonicator for 2 min. Afterwards the solution was rehydrated for 1 hour at room temperature. The size of the liposomes was adjusted by extrusion through 100 nm polycarbonate membrane filter (Whatman Inc., Clifton, NJ) with a Mini-extruder (Avanti Polar Lipids, Alabaster, AL).

Size measurements were performed at room temperature by dynamic light scattering (DLS) using a Zetasizer 3000HS (Malvern Instruments, Herrenburg, Germany)

After size measurements the liposomes were diluted with deionized H₂O to 0.36 mg/ml lipid.

### Preparation of miRNA encapsulated cationic liposome

12 µl miR-26a (4 µM Stock) were added to 120 µl liposome solution (0.36 mg/ml) in water and mixed gently. The miRNA/liposome complexes were incubated for 15 min at room temperature and thereafter the solution was applied to the cell culture multiwell plate containing adipocytes in differentiation medium. The final miRNA concentration in a well was 20 nM.

The results are depicted in Fig. 1. 20I12_1, 20I12_2, 20I12_3 (according to the present invention) show significant improvement of transfection over control whereas comparative examples 08E12, 12E12, 14J11, and 09K11 were within the control range. Comparative results have been obtained according to the general procedures as disclosed herein.

The details of the transfection compositions are also given in the following table

| | Lipid | mg/ml | extrusion | miRNA Endconc. on cells | Lipid Endconc. on cells | Lipid mg absolute on cells | Results: UCP1 (miR-26a:miR-C) | Molar Ratio: cationic: neutral | Ratio: Lipid: miRNA |
|---|---|---|---|---|---|---|---|---|---|
| 20I12_1 | **DOTAP:DOPE 2:1** | 3,6 | EtOH inje. | 22 nM | 27.99 µM | 0,021 | **4,19** | 2:1 | 1300:1 |
| 20I12_2 | **DOTAP:DOPE 2:1** | 3,6 | EtOH inje. | 22 nM | 27.99 µM | 0,021 | **3,64** | 2:1 | 1300:1 |
| 20I12_3 | **DOTAP:DOPE 2:1** | 3,6 | EtOH inje. | 22 nM | 27.99 µM | 0,021 | **5,63** | 2:1 | 1300:1 |
| 08E12 | DOTAP:DOPE **1:1** | 3,6 | EtOH inje. | 22 nM | 27.64 µM | 0,021 | **1,09** | 1:1 | 1300:1 |
| 12E12 | DOTAP:DOPE **3:1** | 3,6 | EtOH inje. | 22 nM | 28.14 µM | 0,021 | **0,99** | 3:1 | 1300:1 |
| 14J11 | **DOTAP** | 2,2 | EtOH inje. | 22.2 nM | 17.47 µM | 0,013 | **1,37** | | 800:1 |
| 09K11 | **DOTAP** | 1,12 | EtOH inje. | 11.1 nM | 8.735 µM | 0,0065 | **1,11** | | 800:1 |

The present invention relates to the following preferred embodiments:
1. A composition for transfecting nucleic acid molecules into eukaryotic cells, in particular into adipocytes, said composition comprising a liposome nanoparticle comprising a cationic lipid and a neutral lipid in a molar ratio of 1.1:1 to 2.9:1 and at least one nucleic acid molecule encapsulated in said liposome nanoparticle.
2. Composition according to embodiment 1, wherein the molar ratio of total lipid to the at least one nucleic acid molecule is 10:1 to 5,000:1.
3. A composition for transfecting nucleic acid molecules into eukaryotic cells, in particular into adipocytes, said composition comprising a liposome nanoparticle comprising a cationic lipid and a neutral lipid and at least one nucleic acid molecule encapsulated in said liposome nanoparticle, wherein the molar ratio of total lipid to the at least one nucleic acid molecule is 10:1 to 5,000:1.
4. Composition according to embodiment 3, wherein the molar ratio of the cationic lipid and the neutral lipid is 1.1:1 to 2.9:1.
5. Composition according to any one of embodiments 1 to 4, wherein the cationic lipid is selected from the group consisting of 1,2-dioleoyl-3-trimethylammonium-propane (DOTAP), Dimethyldioctadecylammonium (DDAB), 1.2-di-O-octadecenyl-3-trimethylammonium propane (DOTMA), 3ß-[N-(N',N'-dimethylaminoethane)-carbamoyl]cholesterol hydrochloride (DC-CHOL), 1,2-dioleoyl-3-dimethylammonium-propane (DODAP), cetyl trimethyl ammonium bromide (CTAB), 1,3-dioleoyloxy-2-(6-carboxy-spermyl)-propylamide (DOSPER), and 1,2-dioleoyl-sn-glycero-3-[(N-(5-amino-1-carboxypentyl)iminodiacetic acid)succinyl](DOGS).
6. Composition according to any one of embodiments 1 to 5, wherein the neutral lipid is selected from the group consisting of dioleoylphosphatidylethanolamine (DOPE), phospholipid 1,2-diphytanoyl-sn-glycero-3-phosphoethanolamine (DPhPE), dioleoylphosphatidylcholine (DOPC), 1,2-dihexadecylphosphoethanolamine, 1,2-dilauroylphosphoethanolamine, 1,2-dilinoleoylphosphoethanol-amine, 1,2-dimyristoylphosphoethanolamine, 1,2-dioleoylphosphoethanolamine, 1,2-dipalmitoylphosphoethanolamine, 1,2-distearoylphosphoethanolamine, 1-palmitoyl-2-oleoylphospho-ethanolamine (POPE), 1,2-dipalmitoylphosphoethanolamine-N-[4(p-maleimidephenyl)bu tyla-mide], 1,2-dipalmitoylphosphoethanolamine-N-[3-(2-pyridyldithio)pro pionate], 1,2-dioleoylphosphor-ethanolamine-N-(succinyl) and 1,2-dipalmitoylphosphoethanolamine-N-(succinyl).
7. Composition according to any one of embodiments 1 to 3, wherein the head group of the neutral lipid is modified with maleimidomethyl-CyClohexane-carboxamide(MCC), malei-midophenyl-butyramide (MPP), N-succinyl, ethylene-glycol, N-dodecanyl, thioethanol and N-glutaryl (GA).
8. Composition according to any one of embodiments 1 to 7, wherein the lipids are coated with polyethylene glycol (PEG), N-hydroxylsuccinimide (NHS) functionalized polyethylene glycol or maleimide functionalized polyethylene glycol.
9. Composition according to any one of embodiments 1 to 8, wherein liposome nanoparticle is modified with protamine, hyaluronic acid, methyl-β-cyclodextrin or albumin.
10. Composition according to any one of embodiments 1 to 9, wherein the at least one nucleic acid molecule is a small RNA having preferably a length of 15 to 100 nucleotides.
11. Composition according to embodiment 10, wherein the small RNA is a siRNA or miRNA, preferably a miRNA, or a miRNA precursor.
12. Composition according to any one of embodiments 1 to 11, wherein the at least one nucleic acid molecule induces or upregulates the expression of UCP1.
13. Composition according to embodiment 12, wherein the at least one nucleic acid molecule negatively interferes concomitantly and independently with at least two UCP1-suppressors and/or suppressors of the UCP1-promoting insulin signalling pathway.
14. Composition according to embodiment 12 or 13, wherein the at least one nucleic acid molecule hybridises to the mRNA of a UCP1-suppressor and/or a suppressor of the UCP1-promoting insulin signalling pathway, thereby inducing degradation or preventing translation of said mRNA.
15. Composition according to any one of embodiments 1 to 11, wherein the at least one nucleic acid molecule downregulates the expression of UCP1.
16. Composition according to any one of embodiments 1 to 14, wherein the at least one nucleic acid molecule is selected from the group consisting of:
   (i) a polynucleotide comprising the nucleotide sequence of SEQ ID No. 1;
   (ii) a polynucleotide comprising the nucleotide sequence of SEQ ID No. 2;
   (iii) a polynucleotide comprising the nucleotide sequence of SEQ ID No. 3;
   (iv) a polynucleotide being at least 25% identical to any one of (i) to (iii); and
   (v) a polynucleotide according to (iv), which comprises to nucleotide sequence of SEQ ID No. 4.
17. Composition according to any one of embodiments 1 to 17, wherein the composition is administered to an individual, by intravenous, subcutaneous, intramuscular, inhalation, especially inhalation of aerosols, absorption via the mucosa of the mouth, intraperitoneal or intradermal administration.
18. Composition according to any one of embodiments 12 to 16 for use in treating or preventing diseases or disorders of the energy homeostasis or related diseases or disorders in a mammal.
19. Composition for the use according to embodiment 18, wherein said disease or disorder of the energy homeostasis is selected from the group consisting of obesity, overweight, hyperglycaemia, adiposity and metabolic syndrome if the at least one nucleic acid molecule induces or upregulates the expression of UCP1.
20. Composition for the use according to embodiment 19, wherein said disease or disorder of the energy homeostasis is selected from the group consisting of anorexia and cachexia if the at least one nucleic acid molecule downregulates the expression of UCP1.
21. Method for transfecting eukaryotic cells, in particular adipocytes, with nucleic acid molecules comprising the step of incubating a composition according to any one of embodiments 1 to 17 with eukaryotic cells, in particular adipocytes.

## Claims

1. A composition for transfecting nucleic acid molecules into eukaryotic cells, in particular into adipocytes, said composition comprising a liposome nanoparticle comprising a cationic lipid and a neutral lipid in a molar ratio of 1.1:1 to 2.9:1 and at least one nucleic acid molecule encapsulated in said liposome nanoparticle.

2. Composition according to claim 1, wherein the molar ratio of total lipid to the at least one nucleic acid molecule is 10:1 to 5,000:1.

3. A composition for transfecting nucleic acid molecules into eukaryotic cells, in particular into adipocytes, said composition comprising a liposome nanoparticle comprising a cationic lipid and a neutral lipid and at least one nucleic acid molecule encapsulated in said liposome nanoparticle, wherein the molar ratio of total lipid to the at least one nucleic acid molecule is 10:1 to 5,000:1.

4. Composition according to claim 3, wherein the molar ratio of the cationic lipid and the neutral lipid is 1.1:1 to 2.9:1.

5. Composition according to any one of claims 1 to 4, wherein the cationic lipid is selected from the group consisting of 1,2-dioleoyl-3-trimethylammonium-propane (DOTAP), Dimethyldioctadecylammonium (DDAB), 1,2-di-O-octadecenyl-3-trimethylammonium propane (DOTMA), 3ß-[N-(N',N'-dimethylaminoethane)-carbamoyl]cholesterol hydrochloride (DC-CHOL), 1,2-dioleoyl-3-dimethylammonium-propane (DODAP), cetyl trimethyl ammonium bromide (CTAB), 1,3-dioleoyloxy-2-(6-carboxy-spermyl)-propyl-amide (DOSPER), and 1,2-dioleoyl-sn-glycero-3-[(N-(5-amino-1-carboxypentyl)iminodiacetic acid)succinyl](DOGS).

6. Composition according to any one of claims 1 to 5, wherein the neutral lipid is selected from the group consisting of dioleoylphosphatidylethanolamine (DOPE), phospholipid 1,2-diphytanoyl-sn-glycero-3-phosphoethanolamine (DPhPE), dioleoylphosphatidylcholine (DOPC), 1,2-dihexadecylphosphoethanolamine, 1,2-dilauroylphosphoethanolamine, 1,2-dilinoleoylphosphoethanol-amine, 1,2-dimyristoylphosphoethanolamine, 1,2-dioleoylphosphor-ethanolamine, 1,2-dipalmitoylphosphoethanolamine, 1,2-distearoylphosphoethanolamine, 1-palmitoyl-2-oleoylphospho-ethanolamine (POPE), 1,2-dipalmitoylphosphoethanolamine-N-[4(p-maleimidephenyl)bu tyla-mide], 1,2-dipalmitoylphosphoethanolamine-N-[3-(2-pyridyldithio)pro pionate], 1,2-dioleoylphosphoethanolamine-N-(succinyl) and 1,2-dipalmitoylphosphoethanolamine-N-(succinyl).

7. Composition according to any one of claims 1 to 3, wherein the head group of the neutral lipid is modified with maleimidomethyl-cyclohexane-carboxamide(MCC), malei-midophenyl-butyramide (MPP), N-succinyl, ethylene-glycol, N-dodecanyl, thioethanol and N-glutaryl (GA).

8. Composition according to any one of claims 1 to 7, wherein the lipids are coated with polyethylene glycol (PEG), N-hydroxylsuccinimide (NHS) functionalized polyethylene glycol or maleimide functionalized polyethylene glycol.

9. Composition according to any one of claims 1 to 8, wherein the at least one nucleic acid molecule is a small RNA having preferably a length of 15 to 100 nucleotides, especially wherein the small RNA is a siRNA or miRNA, preferably a miRNA, or a miRNA precursor.

10. Composition according to any one of claims 1 to 9, wherein the at least one nucleic acid molecule induces or upregulates the expression of UCP1, preferably wherein the at least one nucleic acid molecule negatively interferes concomitantly and independently with at least two UCP1-suppressors and/or suppressors of the UCP1-promoting insulin signalling pathway, especially wherein the at least one nucleic acid molecule hybridises to the mRNA of a UCP1-suppressor and/or a suppressor of the UCP1-promoting insulin signalling pathway, thereby inducing degradation or preventing translation of said mRNA.

11. Composition according to any one of claims 1 to 9, wherein the at least one nucleic acid molecule downregulates the expression of UCP1.

12. Composition according to any one of claims 1 to 11, wherein the at least one nucleic acid molecule is selected from the group consisting of:
(i) a polynucleotide comprising the nucleotide sequence of SEQ ID No. 1;
(ii) a polynucleotide comprising the nucleotide sequence of SEQ ID No. 2;
(iii) a polynucleotide comprising the nucleotide sequence of SEQ ID No. 3;
(iv) a polynucleotide being at least 25% identical to any one of (i) to (iii); and
(v) a polynucleotide according to (iv), which comprises to nucleotide sequence of SEQ ID No. 4.

13. Composition according to any one of claims 1 to 12 for use in treating or preventing diseases or disorders of the energy homeostasis or related diseases or disorders in a mammal, preferably wherein said disease or disorder of the energy homeostasis is selected from the group consisting of obesity, overweight, hyperglycaemia, adiposity and metabolic syndrome if the at least one nucleic acid molecule induces or upregulates the expression of UCP1, especially wherein said disease or disorder of the energy homeostasis is selected from the group consisting of anorexia and cachexia if the at least one nucleic acid molecule downregulates the expression of UCP1.

14. Method for transfecting eukaryotic cells, in particular adipocytes, with nucleic acid molecules comprising the step of incubating a composition according to any one of claims 1 to 12 with eukaryotic cells, in particular adipocytes.
